# EUROPEAN PATENT SPECIFICATION

(11) **EP 2 257 215 B1**
(45) Date of publication and mention of the grant of the patent: **11.11.2015**
(21) Application number: 09706342.4
(22) Date of filing: 30.01.2009
(51) Int. Cl.: A61B 5/00, A61M 25/00, B01D 61/28

(54) **A MICRO-DIALYSIS PROBE AND A METHOD OF MAKING THE PROBE**
MIKRODIALYSESONDE UND VERFAHREN ZUR HERSTELLUNG DER SONDE
SONDE DE MICRODIALYSE ET PROCÉDÉ DE FABRICATION DE LA SONDE

(30) Priority: 30.01.2008 DK 200800121
(43) Date of publication of application: 08.12.2010
(73) Proprietor: Flowsion A/S, 6400 Sønderborg (DK)
(72) Inventor: TRÖSKEN, Volker, 58456 Witten (DE); STENSTRØM, Theiss, 6400 Sønderborg (DK); DIRAC, Holger, 3460 Birkerød (DK)
(74) Representative: Inspicos A/S
(86) International application number: PCT/DK2009/000023
(87) International publication number: WO 2009/095020

(56) References cited:
- WO-A-01/06928
- WO-A-99/45982
- GB-A- 2 130 916
- GB-A- 2 442 209

## Description

### FIELD OF THE INVENTION

The present invention relates to a micro-dialysis probe for collecting substances of interest, e.g. ions or molecules, in particular from human or animal fluids or tissue. The micro-dialysis probe is of a kind having one or more flow channels carrying a perfusion fluid, the flow channel(s) being in contact with the tissue via one or more semi-permeable membranes. The substances of interest pass through the membrane(s) and are carried away by the perfusion liquid for further analysis.

The present invention further relates to a method of making such a micro-dialysis probe in an easy and cost effective manner.

### BACKGROUND OF THE INVENTION

US 6,346,090 discloses a catheter for insertion into a blood vessel of a living being, which includes an elongated catheter body and membrane defining a dialysis chamber. The dialysis chamber comprises a plurality of tubular members, each having an inlet end and an outlet end. All the inlet ends are connected to a first channel provided for delivery of dialysis liquid to the dialysis chamber. All the outlet ends are connected to a second channel provided for the discharge of dialysis liquid from the dialysis chamber. The tubular members are preferably semi-permeable membranes, the interiors of the tubular members together forming the dialysis chamber.

US 7,008,398 discloses a micro-dialysis probe extending longitudinally between a proximal probe opening and a distal probe tip and having a supply line and a drainage line for a drip-feed solution A tube may be provided for supporting the drainage line. A dialysis section, wherein the flow channel for the drip-feed solution experiences an inversion, is formed generally between the supply line and the drainage line, in the vicinity of the probe tip. The drainage line may consist of a dialysis hollow fibre, preferably formed to be replaceable, and correspondingly sealed in, the tube and in particular its supporting section comprising recesses via which the hollow fibre is exposed outwards, to be able to perform dialysis.

WO 99/45982 (closest prior art) discloses a catheter to be inserted into and guided by a blood vessel. The catheter comprises an elongate catheter body, having a distal end and a proximal end, and includes at least two channels. A first and a second channel for micro-dialysis solution are connected with each other so that micro-dialysis solution can flow from one channel to the other. An opening is provided in the catheter body, and a micro-dialysis membrane tube is arranged around the catheter body in such a manner that it covers the opening.

There is a need to provide a micro-dialysis probe which is easy and cost effective to manufacture, and which is suitable for mass production. Furthermore, there is a need for a micro-dialysis probe which is capable of collecting a concentration of substances of interest which is representative of a local concentration at the position of the probe.

### SUMMARY OF THE INVENTION

It is, thus, an object of the invention to provide a method of making a micro-dialysis probe, the method being easier and more cost effective to perform than similar prior art methods.

It is a further object of the invention to provide a method of making a micro-dialysis probe, the method being suitable for mass production and for automated manufacturing processes.

It is an even further object of the invention to provide a micro-dialysis probe which can be manufactured in an easy and cost effective manner.

According to a first aspect of the invention the above and other objects are fulfilled by providing a method of making a micro-dialysis probe, the method comprising the steps of:
- providing a first flow channel, a second flow channel and a separating wall arranged in such a manner that it separates the first flow channel and the second flow channel,
- providing at least one opening in an outer wall of the first flow channel or the second flow channel,
- arranging a semi-permeable membrane tube in each opening, and
- removing material from the separating wall, thereby providing a communication opening providing fluid communication between the first flow channel and the second flow channel.

In the present context the term 'micro-dialysis probe' should be interpreted to mean a probe which is adapted to collect substances of interest, in particular from human or animal tissue, such as blood, by means of dialysis, i.e. the substances of interest diffuse through a semi-permeable membrane to be collected by a perfusion fluid flowing in an interior part of the probe. Furthermore, the probe has dimensions which are sufficiently small to at least substantially prevent turbulence in a fluid flowing in the probe.

The first flow channel and the second flow channel may be arranged relative to each other in any suitable manner, as long as they are separated by the separating wall. Thus, the separating wall interfaces an interior part of the first flow channel as well as an interior part of the second flow channel. The first flow channel and the second flow channel are preferably adapted to have a perfusion fluid flowing therein.

At least one opening is provided in an outer wall of the first flow channel or the second flow channel, and a semi-permeable membrane tube is arranged in each opening. Accordingly, access is provided to an interior part of the first flow channel and/or the second flow channel via a wall of a semi-permeable membrane tube. Accordingly, substances of interest can diffuse into one or both of the flow channels via the semi-permeable membrane tubes, and the opening(s) with tube(s) arranged therein may thereby form dialysis regions.

In the present context the term 'semi-permeable' should be interpreted to mean that some substances are allowed to pass the wall of the membrane tube while other substances are not allowed to pass. Preferably, the membrane tubes allow substances of interest, such as glucose, to pass, while perfusion liquid flowing in the flow channels is substantially not allowed to pass.

The fact that a semi-permeable membrane tube is arranged in each opening, rather than semi-permeable membrane being arranged circumferentially to the probe and along an exterior part of the probe, introduces the following advantages. The method is suitable for being performed in an automated process, since it is not necessary to pull the relatively fragile membrane tubes over the probe, and since it is possible to automatically position the membrane tube(s) in the opening(s). Furthermore, this arrangement of the membrane tube(s) opens the possibility of positioning a wall part of a membrane tube at a distance from a wall part of the probe, thereby preventing the membrane wall from getting into contact with firm tissue, such as a wall of a blood vessel having the probe inserted therein. This will be described further below.

Generally the method according to the first aspect of the invention is very suitable for automated manufacturing processes, because the flow channels with the separating wall can be manufactured as long, such as in the range of several metres or even in the range of kilometre, tubes in a standard process, such as extrusion. The long tube may then be cut into pieces of a desired length, and these pieces can subsequently be processed using standard processing methods, such as mechanical drilling, laser cutting or other suitable cutting processes. Finally, the membrane tubes can also be manufactured in long tubes which are subsequently cut into pieces of desired length and arranged in the openings of the probe.

The method comprises the step of removing material from the separating wall in order to provide a communication opening. The communication opening provided in this manner provides fluid communication between the first flow channel and the second flow channel. Perfusion fluid may, thus, pass from the first flow channel to the second flow channel, or vice versa, via the communication opening. This may cause a reversal of flow direction of the perfusion fluid in such a manner that the flow direction on one side of the separating wall is substantially reversed relatively to the flow direction on the opposing side of the separating wall. Thereby one flow channel may be used for advancing perfusion fluid to a sampling site, while the other flow channel may be used for returning perfusion fluid carrying collected substances of interest to an analysis apparatus, or for autosampling, or for collection, e.g. in a vial or other suitable container, for further analysis at a later time.

It is an advantage that the communication opening is provided simply by removing material from the separating wall, because this is a very easy process. Furthermore, this makes the manufacturing process suitable for mass production, since the flow channels and the separating wall can be manufactured as standard components, e.g. in the form of long tubes which can be cut to a desired length. Material is then removed from the separating wall to form the communication opening.

The step of providing at least one opening in an outer wall may comprise providing a first opening in an outer wall of the first flow channel and providing a second opening in an outer wall of the second flow channel. According to this embodiment at least two openings having a semi-permeable membrane tube arranged therein are provided, i.e. at least one in an outer wall of the first flow channel and at least one in an outer wall of the second flow channel. Increasing the number of openings having a semi-permeable membrane tube arranged therein correspondingly increases the area of semi-permeable membrane being in contact with the tissue where the sample is collected. Thereby an improved sampling can be obtained. Furthermore, arranging a membrane tube in each of the flow channels reduces the risk of both membrane tubes being blocked, e.g. because the membrane tube is arranged close to a wall of a blood vessel having the probe arranged therein, as compared to a situation where only one of the flow channels has membrane tube(s) arranged therein.

The step of arranging a semi-permeable membrane tube may be performed in such a manner that the semi-permeable membrane tube defines an outer surface which is arranged closer to an interior part of the flow channel than the outer wall of the flow channel having the opening formed therein, and in such a manner that the semi-permeable membrane tube and the outer wall thereby in combination define a sample collecting volume. According to this embodiment, the outer wall of the semi-permeable membrane tube is 'lowered' relatively to the outer wall in which the corresponding opening is provided. This is particularly an advantage when the probe is used for sampling substances of interest from a blood vessel of a human or animal. During such sampling it may occur that the probe is positioned relatively close to a wall of the blood vessel. If the sampling takes place from such a position, there is a risk that the concentration of collected substances of interest does not reflect the actual local concentration in the blood. By positioning the semi-permeable membrane tube as described above it is ensured that, regardless of how close the probe is positioned to a wall of the blood vessel, the semi-permeable membrane tube is always separated a minimum distance from the wall of the blood vessel, the minimum distance being defined by the distance between the outer wall of the semi-permeable membrane tube and the outer wall of the flow channel. Thereby it can be ensured that the collected concentration of substances of interest corresponds to the actual concentration of these substances in the blood.

The step of arranging a semi-permeable membrane tube may comprise gluing a part of the semi-permeable membrane tube to an edge of the opening formed in the outer wall of the flow channel. According to this embodiment, the semi-permeable membrane tube is fixed to the opening formed in the outer wall of the flow channel. Furthermore, by choosing a suitable glue and performing the gluing operation carefully, it can be ensured that the only passage between an interior part of the flow channel and the exterior of the flow channel is via the semi-permeable membrane. Thereby it is ensured that only substances which are allowed to pass the semi-permeable membrane enter the flow channel.

The method may further comprise the step of inserting a plug at an end part of the micro-dialysis probe, in such a manner that the plug forms a wall of the communication opening. According to this embodiment, the communication opening has preferably been formed by removing material from an end part of the probe. Afterwards, the first flow channel and the second flow channel are sealed by means of the plug, while leaving the communication opening open. This is a very easy manner of manufacturing a micro-dialysis probe, and it is particularly suitable for mass production, since the flow channels and the separating wall may, in this case, advantageously be manufactured in the form of long tubes which are subsequently cut to a desired length. The plug may simply be formed by glue.

The method may further comprise the steps of:
- providing a semi-permeable membrane tube,
- applying a layer of substantially non-permeable material onto selected regions of an outer surface of the semi-permeable membrane tube, and
- cutting the semi-permeable membrane tube at positions corresponding to the regions having a layer of substantially non-permeable material applied thereto, thereby forming at least one segment of semi-permeable membrane tube,
and the step of arranging a semi-permeable membrane tube in each opening may comprise arranging at least one of said segment(s) of semi-permeable membrane tube in an opening.

In the present context the term 'substantially non-permeable material' should be interpreted to mean a material which does not allow substances which are expected to be found in a region where it is intended to position the probe to pass, or only allows such passage to a very limited extent. In particular, substances of interest as defined above should not be allowed to pass through the walls of the membrane tube at the regions having the layer of non-permeable material applied thereto. Accordingly, such substances are only allowed to pass through the walls of the membrane tube via the regions which do not have a layer of non-permeable material applied thereto.

Thus, according to this embodiment the semi-permeable membrane tube which is arranged in the opening of the first/second flow channel has at least one surface region which is not permeable and at least one surface region which is semi-permeable, and substances of interest are only allowed to pass the walls of the membrane tube via the semi-permeable region(s). By carefully designing the regions having a layer of substantially non-permeable material applied thereto, and thereby also the regions which do not have such a layer, it is possible to control the area, shape and position of the part of the membrane which allows substances of interest to pass. This is advantageous for the following reasons.

In most cases the micro-dialysis probe being manufactured by the method according to the invention is very small. There is therefore a risk that when a semi-permeable membrane tube is arranged in and attached to an opening, attaching material, such as glue, is applied to surface parts of the semi-permeable membrane tube. Since such attaching material most likely has diffusion properties which differ from the diffusion properties of the material of the semi-permeable membrane tube, this will affect the overall diffusion properties of the membrane tube. Furthermore, it is often very difficult to control the attaching process, mainly due to the size of the probe, and the diffusion properties of the membrane tube therefore become unpredictable, thereby rendering the operation of the resulting probe unpredictable, and it is therefore necessary to perform individual calibration of each probe in order to ensure that the measurements performed using the probe are reliable. By controlling the area, shape and position of the semi-permeable part of the membrane tube as described above, these problems are avoided, or at least significantly reduced, in particular if the non-permeable parts are arranged at positions where the membrane tube is attached to the opening.

The step of applying a layer of substantially non-permeable material may be performed by means of a chemical vapour deposition (CVD) process, by means of a physical deposition process, e.g. a sputtering process, by means of an evaporation process, e.g. an electron beam evaporation process, or simply by means of spraying.

Alternatively or additionally, the step of applying a layer of substantially non-permeable material may comprise applying a layer of biocompatible material, preferably titanium (Ti) or an alloy of titanium. As an alternative, the substantially non-permeable material may be tantalum (Ta) or an alloy of tantalum, silver (Ag) or an alloy of silver, gold (Au), or a biocompatible polymer.

Alternatively or additionally, the step of applying a layer of substantially non-permeable material may comprise arranging a mask at positions corresponding to regions of the semi-permeable membrane tube which are not to have the layer of substantially non-permeable material applied thereto. When the mask is in position, the substantially non-permeable material may be applied to the masked surface, e.g. by means of a CVD process or another suitable process, the mask ensuring that the material is not applied to the masked regions.

According to a second aspect of the invention the above and other objects are fulfilled by providing a micro-dialysis probe comprising:
- a flow tube comprising:
   - a first flow channel,
   - a second flow channel, and
   - a separating wall arranged in such a manner that it separates the first flow channel and the second flow channel,
- at least one semi-permeable membrane tube, each semi-permeable membrane tube being arranged in an opening formed in an outer wall of the first flow channel or the second flow channel, and
- a communication opening defined in the separating wall for providing fluid communication between the first flow channel and the second flow channel.

The micro-dialysis probe according to the second aspect of the invention is preferably manufactured using the method according to the first aspect of the invention. The advantages described above are thereby obtained by the micro-dialysis probe.

It should be noted that a person skilled in the art would readily recognise that any feature described in combination with the first aspect of the invention could also be combined with the second aspect of the invention, and vice versa.

The flow channels and/or the separating wall is/are preferably made from a biocompatible material, e.g. a biocompatible polymer or Teflon^{®}.

The semi-permeable membrane tube may preferably comprise polymers, such as polyethersulfone or polyvinylpyrrolidone, or a polyether-polycarbonate block copolymer. It may, e.g., be based on polyvinylidene fluoride (PVDF) and a hydrophilic polymer, such as copolymers of acrylonitrile or monomers containing sulphonic groups.

The micro-dialysis probe may comprise at least a first semi-permeable membrane tube arranged in an opening formed in an outer wall of the first flow channel and a second semi-permeable membrane tube arranged in an opening of the second flow channel. As described above, the total area of semi-permeable membrane in contact with the tissue from which the sampling takes place can be increased, and the risk that both of the membrane tubes are blocked is reduced.

The communication opening may be arranged at an end portion of the probe. By positioning the communication opening in this manner it is possible to manufacture the micro-dialysis probe in a very simple manner, because it is easier to process an end portion of the separating wall than another part of the separating wall.

The communication opening may advantageously have been formed by removing material from an end portion of the separating wall. As described above, the probe is thereby very easy to manufacture. Furthermore, this may be used to control the flow rate of the probe in the following manner. The amount of material removed from the separating wall determines the cross sectional dimensions of the communication opening interconnecting the first flow channel and the second flow channel, and thereby the maximum flow rate in this part of the flow channel. Accordingly, this step may be used to ensure that various probes define substantially identical, or at least similar, flow rates..

At least one of the semi-permeable membrane tubes may define an outer surface which is arranged closer to an interior part of the flow channel than the outer wall of the flow channel having the opening formed therein, the semi-permeable membrane tube and the outer wall thereby in combination defining a sample collecting volume. As described above, designing the probe in this manner ensures that the concentration of substances of interest collected by the probe reflects the actual local concentration of these substances in the tissue where the sampling takes place.

The micro-dialysis probe may further comprise a plug for sealing the first flow channel and the second flow channel, said plug forming a wall of the communication opening. This has also been described above.

The separating wall may be a substantially plane wall dividing an interior part of the flow tube into the first flow channel and the second flow channel. Normally, the remaining walls of the flow channels will not be substantially plane, but will rather be curved, such as concave, e.g. forming part of a substantially cylindrical tube, e.g. having a circular or oval cross section. The micro-dialysis probe according to this embodiment has a so-called 'double D' structure, which is a very simply manner of providing a flow system with two separate flow channels. This structure has the advantage that the strength of the outer walls of the probe, and thereby of the probe construction as such, is very high. As an alternative, the flow channels may be formed in any other suitable manner, and they may be arranged relative to each other in any other suitable manner. For instance, each flow channel may be formed by a separate tube, the tubes being arranged adjacent to each other or one inside the other.

According to one embodiment at least one of the semi-permeable membrane tube(s) may comprise at least one surface region having a layer of substantially non-permeable material applied thereto. As described above, it is thereby possible to control the free membrane area, and thereby affect the overall diffusion properties of the membrane tube(s). It is, furthermore, possible to ensure the presence of an area where the membrane can be glued or welded into the openings of the flow channels.

At least one of the semi-permeable membrane tube(s) may comprise two surface regions having a layer of substantially non-permeable material applied thereto, said surface regions being arranged in such a manner that a surface region without substantially non-permeable material applied thereto is arranged between said two surface regions. According to this embodiment, the membrane tube can easily be arranged in the opening in such a manner that the surface regions having a layer of substantially non-permeable material applied thereto form interfaces between the edges of the opening and the membrane tube, while the surface region without substantially non-permeable material applied thereto, i.e. the semi-permeable part of the membrane tube, is not arranged in contact with the edges of the opening.

The substantially non-permeable material may be a biocompatible material, preferably titanium (Ti) or an alloy of titanium. Alternatively, the biocompatible material may be tantalum (Ta) or an alloy of tantalum, silver (Ag) or an alloy of silver, gold (Au), or a biocompatible polymer.

### BRIEF DESCRIPTION OF THE DRAWINGS

The invention will now be described in further detail with reference to the accompanying drawings in which
Fig. 1 is a perspective view of a flow tube for use in a micro-dialysis probe according to an embodiment of the invention,
Fig. 2 is a cross sectional view of the flow tube of Fig. 1,
Fig. 3 is a cross sectional view of a micro-dialysis probe according to an embodiment of the invention, during manufacture,
Fig. 4 is a cross sectional view of the micro-dialysis probe of Fig. 3 after completion,
Fig. 5 is a cross sectional view of part of the micro-dialysis probe of Figs. 3 and 4 during use,
Fig. 6 is a cross sectional view of a micro-dialysis probe according to a second embodiment of the invention,
Fig. 7 is a schematic drawing illustrating preparation of a semi-permeable membrane tube using a method according to an embodiment of the invention,
Fig. 8 is a side view of a semi-permeable membrane tube for use in a micro-dialysis probe according to a third embodiment of the invention,
Fig. 9 is a cross sectional view of a micro-dialysis probe provided with two semi-permeable membrane tubes of the kind shown in Fig. 8, and
Fig. 10 is a cross sectional view of a micro-dialysis probe according to a fourth embodiment of the invention.

### DETAILED DESCRIPTION OF THE DRAWINGS

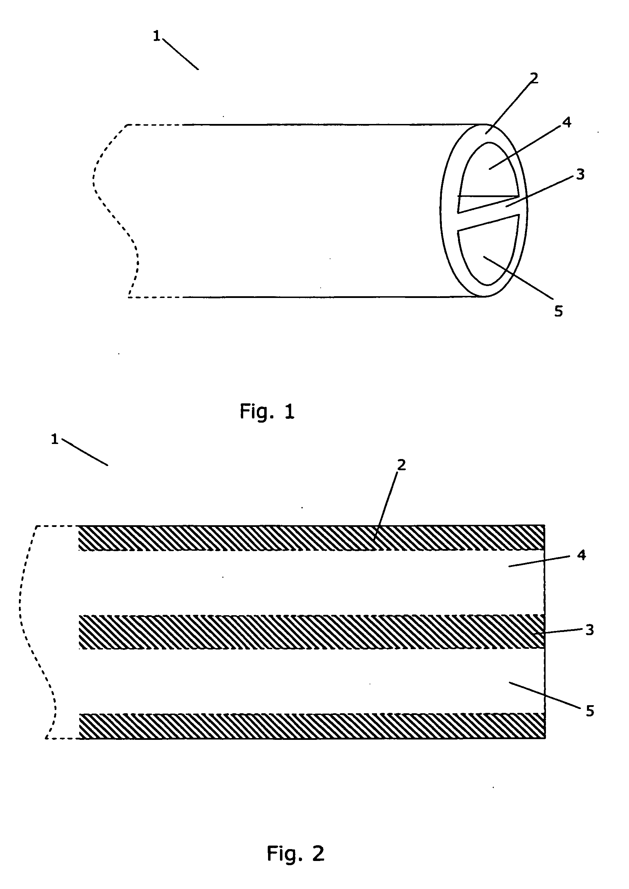

Fig. 1 is a perspective view of a flow tube 1 for use in a micro-dialysis probe according to an embodiment of the invention. The flow tube 1 of Fig. 1 comprises an outer wall 2 defining a substantially circular cross section, and a separating wall 3 dividing the interior of the flow tube 1 into a first flow channel 4 and a second flow channel 5. Thus, the flow tube of Fig. 1 has a so-called 'double D' shape.

Fig. 2 is a cross sectional view of the flow tube 1 of Fig. 1. The separating wall 3 and the flow channels 4, 5 are visible.

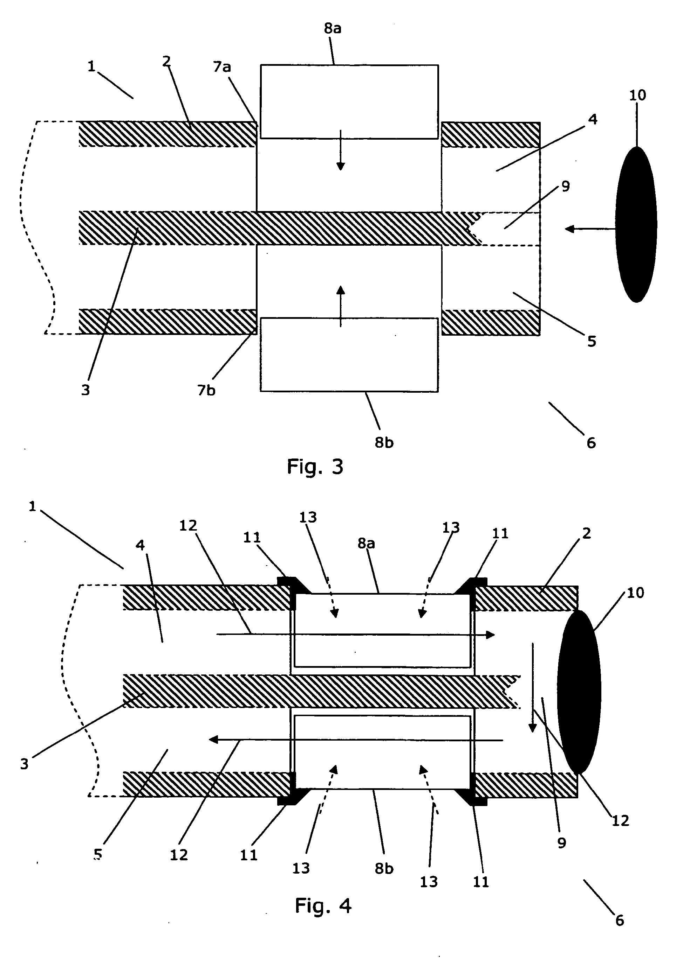

Fig. 3 is a cross sectional view of a micro-dialysis probe 6 according to an embodiment of the invention during manufacture. The micro-dialysis probe 6 is manufactured from a flow tube 1 of the kind shown in Figs. 1 and 2. In Fig. 3 two recesses 7 have been provided in the outer wall 2 of the flow tube 1, one recess 7a providing communication to the first flow channel 4 and another recess 7b providing communication to the second flow channel 5. Two semi-permeable membrane tubes 8a, 8b are in the process of being positioned in the recesses 7a, 7b.

A portion of the separating wall 3 has been removed near an end of the flow tube 1. Thereby a communication opening 9 has been formed between the first flow channel 4 and the second flow channel 5, through the separating wall 3. A plug 10 is in the process of being positioned at the end portion of the flow tube 1, in order to provide sealing for the first flow channel 4 and the second flow channel 5, while maintaining the communication opening 9, thereby allowing fluid to flow from the first flow channel 4 to the second flow channel 5, or vice versa, via the communication opening 9.

Simply removing material from an end portion of the separating wall 3, and subsequently inserting a sealing plug 10, is a very simple manner of providing the communication opening 9. Accordingly, this allows the micro-dialysis probe to be manufactured in an easy and cost effective manner. Furthermore, long tubes of the kind shown in Figs. 1 and 2 can easily be manufactured, and suitable pieces can be cut off and processed as described above to make a micro-dialysis probe 6. This makes the probe 6 and the manufacturing process suitable for mass production and for automated processes.

Fig. 4 is a cross sectional view of the micro-dialysis probe 6 of Fig. 3. It can be seen that the semi-permeable membrane tubes 8a, 8b have been attached to the outer wall 2 by means of a suitable glue 11. It can also be seen that the plug 10 is attached at the end of the flow tube 1 in such a manner that is seals the flow channels 4, 5, while maintaining the communication opening 9.

Perfusion fluid flows through the micro-dialysis probe 6 as illustrated by arrows 12, i.e. into the first flow channel 4, through the communication opening 9, into the second flow channel 5 leading the perfusion fluid out of the micro-dialysis probe 6. Accordingly, the perfusion fluid passes both of the semi-permeable membrane tubes 8a, 8b, and substances of interest may thereby diffuse across either of the semi-permeable membrane tubes 8a, 8b, as indicated by arrows 13, to be collected by the perfusion fluid.

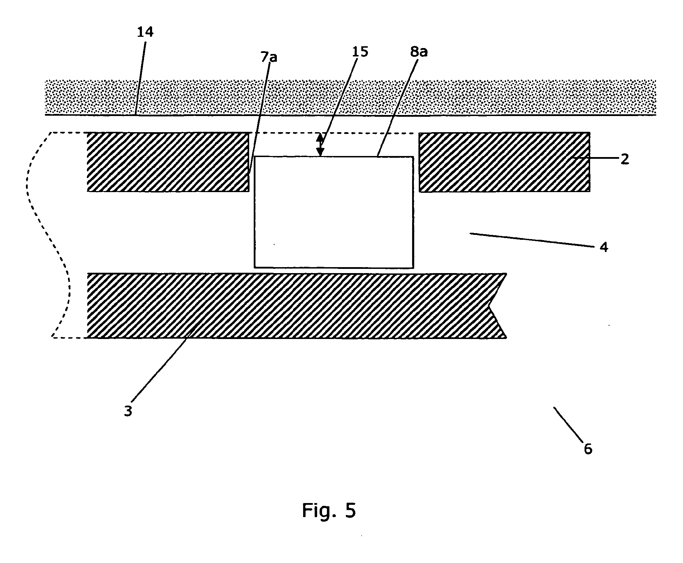

Fig. 5 is a cross sectional view of part of the micro-dialysis probe 6 of Figs. 3 and 4, during operation. The micro-dialysis probe 6 is positioned at a collecting position, e.g. inserted into a blood vessel, such a as a vein or an artery. A wall 14 of the vessel can be seen.

It is clear from Fig. 5 that the semi-permeable membrane tube 8a is arranged in the recess 7a in such a manner that a distance, indicated by arrow 15, is defined between an outer surface of the outer wall 2 and an outer surface of the semi-permeable membrane tube 8a. Thereby it is ensured that, regardless of how close the outer surface of the outer wall 2 is positioned to the wall 14 of the vessel, the outer wall of the semi-permeable membrane tube 8a will always be positioned at least at a certain distance from the wall 14 of the vessel. If the semi-permeable membrane tube 8a is positioned too close to the wall 14, there is a risk that the sampling of substances of interest collected through the semi-permeable membrane tube 8a is not representative of the actual local concentration of the substances of interest in the blood where the sampling takes place. Thus, the micro-dialysis probe 6 shown in Fig. 5 ensures that a sampling which is representative of the local concentration of substances of interest in the blood can be obtained.

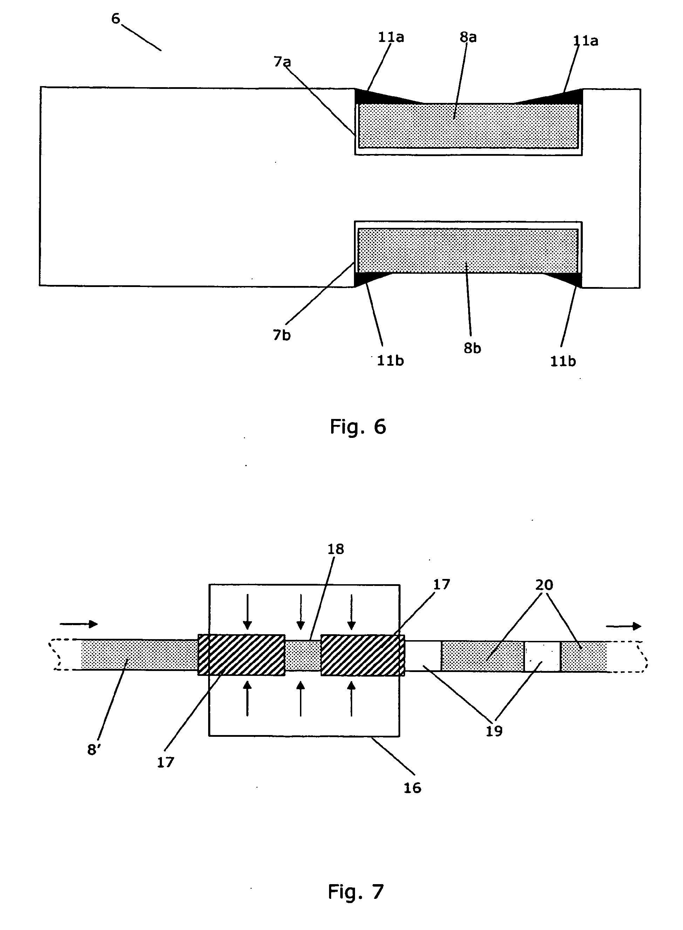

Fig. 6 is a cross sectional view of a micro-dialysis probe 6 according to a second embodiment of the invention. The micro-dialysis probe 6 operates similarly to the probe shown in Fig. 4, and this will therefore not be described in further detail here. For the sake of clarity the flow channels and the separating wall are not shown in Fig. 6.

In the micro-dialysis probe 6 of Fig. 6 semi-permeable membrane tubes 8a, 8b have been arranged in recesses 7a, 7b as described above, and attached by means of glue 11a, 11b. It is clear from Fig. 6 that the area of the first semi-permeable membrane tube 8a covered by glue 11 a is significantly larger than the corresponding area of the second semi-permeable membrane tube 8b covered by glue 11 b. It must be expected that the glue 11 a, 11 b affects the diffusion properties of the semi-permeable membrane tube 8a, 8b. Accordingly, the size of the area covered by glue 11 a, 11 b must be expected to affect the overall diffusion properties of the semi-permeable membrane tube 8a, 8b, and thereby the operation of the micro-dialysis probe 6. Thus, in the probe 6 shown in Fig. 6 the diffusion properties of the first semi-permeable membrane tube 8a differ from the diffusion properties of the second semi-permeable membrane tube 8b, even though the tubes 8a, 8b and the recesses 7a, 7b are identical. As a consequence, the overall diffusion properties of the micro-dialysis probe 6 are unpredictable, and it is therefore necessary to perform an individual calibration of the probe 6 in order to ensure reliable operation.

Fig. 7 is a schematic drawing illustrating preparation of a semi-permeable membrane tube 8' using a method according to an embodiment of the invention. The semi-permeable membrane tube 8' is arranged in a deposition chamber 16 and masks 17 are positioned in such a manner that selected parts are masked while other selected parts 18 are exposed. A layer of substantially non-permeable material, e.g. tantalum (Ta) or another suitable material, is then deposited onto the un-masked parts 18, thereby producing first surface regions 19 of the semi-permeable membrane tube 8' being substantially non-permeable as well second surface regions 20 being semi-permeable. Subsequently the membrane tube 8' may be cut into suitable membrane tubes 8 which can be arranged in a recess of a flow channel as described above.

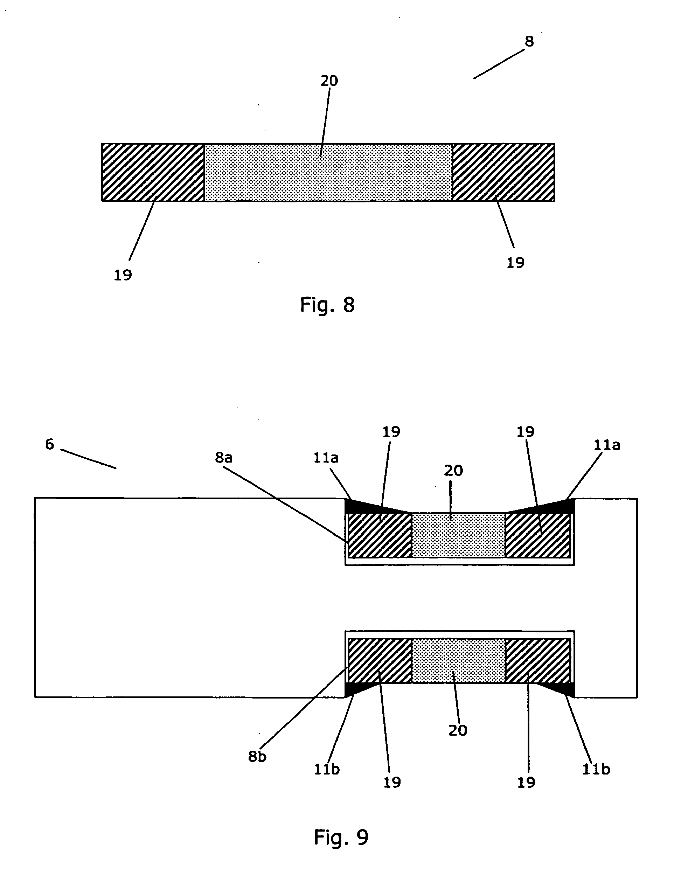

Fig. 8 shows a semi-permeable membrane tube 8 which has been cut from a membrane tube 8' of the kind shown in Fig. 7. It is clear from Fig. 8 that the semi-permeable membrane tube 8 is provided with first surface regions 19 being substantially non-permeable and a second surface region 20 being semi-permeable. The membrane tube 8 may advantageously be used for a micro-dialysis probe according to the invention.

Fig. 9 is a cross sectional view of a micro-dialysis probe 6 having two semi-permeable membrane tubes 8a, 8b of the kind shown in Fig. 8 arranged in respective recesses 7a, 7b of flow channels (not shown). The membrane tubes 8a, 8b are attached to the recesses 7a, 7b by means of glue 11a, 11b. It is clear that, even though the glue 11a used for attaching the first membrane tube 8a covers a significantly larger area than the glue 11b used for attaching the second membrane tube 8b, the semi-permeable part 20 of each of the membrane tubes 8a, 8b remains unaffected. Accordingly, the overall diffusion properties of the membrane tubes 8a, 8b are substantially unaffected by the glue 11 a, 11 b, in particular by the area covered by the glue 11a, 11 b. This is very advantageous because the predictability of the overall diffusion properties of the probe 6 is thereby significantly enhanced.

It should be noted that the substantially non-permeable surface regions 19 may be provided in other ways than the one described above. For instance, the semi-permeable membrane tube may undergo a plasma treatment which causes pores of the membrane to close. Subsequently, the treated areas may be painted in order to provide visual identification of the treated areas. Thereby an operator performing the subsequent gluing operation is able to see where to apply the glue. The important feature is that substantially non-permeable areas are provided, and that these are visually identifiable.

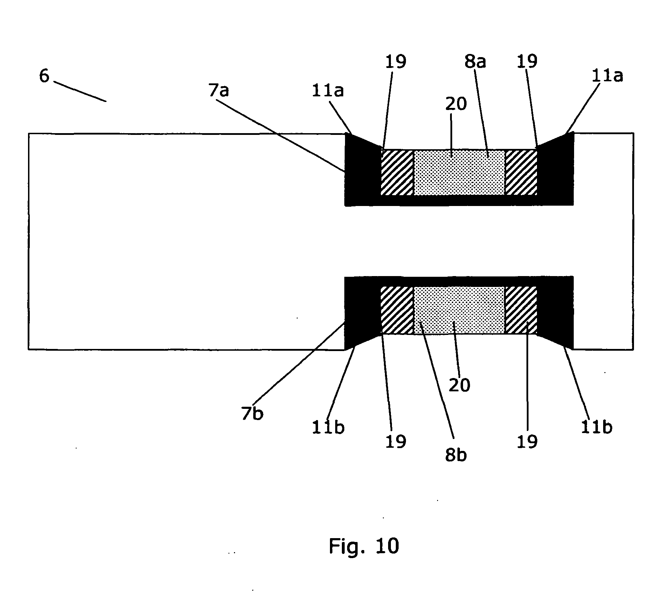

Fig. 10 is a cross sectional view of a micro-dialysis probe 6 according to a fourth embodiment of the invention. The micro-dialysis probe 6 of Fig. 10 operates essentially as the probes shown in Figs. 4 and 6, and this will therefore not be described in further detail here. For the sake of clarity the flow channels and the separating wall are not shown in Fig. 10.

In the micro-dialysis probe 6 of Fig. 10 the semi-permeable membrane tubes 8a, 8b have been arranged in the recesses 7a, 7b as described above and attached by means of glue 11 a, 11 b. It can be seen that the glue 11 a, 11 b covers the entire interface between a recess 7a, 7b and a corresponding semi-permeable membrane tube 8a, 8b. Thereby it can be ensured that the only access between the flow channels and the exterior of the probe 6 is via one of the semi-permeable membrane tubes 8a, 8b, since the glue 11 a, 11 b seals any gaps which may be present between a recess 7a, 7b and the corresponding membrane tube 8a, 8b. Such gaps may result from the membrane tube 8a, 8b not fully filling out the entire flow channel, and they are undesirable because they may allow impurities, which are not allowed to pass the semi-permeable membrane tubes 8a, 8b, to enter the flow channels.

## Claims

1. A micro-dialysis probe (6) comprising:
- a flow tube (1) comprising:
- a first flow channel (4),
- a second flow channel (5), and
- a separating wall (3) arranged in such a manner that it separates the first flow channel (4) and the second flow channel (5),
- a communication opening (9) defined in the separating wall (3) for providing fluid communication between the first flow channel (4) and the second flow channel (5); **characterised by**
- at least one semi-permeable membrane tube (8), each semi-permeable membrane tube (8) being arranged in an opening (7) formed in an outer wall (2) of the first flow channel (4) or the second flow channel (5), and
wherein each semi-permeable membrane tube (8) comprises at least one non-permeable section (19).

2. A micro-dialysis probe (6) according to claim 1, wherein each non-permeable section (19) forms the area where the semi-permeable membrane (8) is glued or welded into the opening (7).

3. A micro-dialysis probe (6) according to claim 2, comprising at least a first semi-permeable membrane tube (8a) arranged in an opening (7a) formed in an outer wall (2) of the first flow channel (4) and a second semi-permeable membrane tube (8b) arranged in an opening (7b) of the second flow channel (5).

4. A micro-dialysis probe (6) according to claim 2 or 3, wherein the communication opening (9) is arranged at an end portion of the probe (6).

5. A micro-dialysis probe (6) according to claim 4, wherein the communication opening (9) has been formed by removing material from an end portion of the separating wall (3).

6. A micro-dialysis probe (6) according to any of claims 1-15, wherein at least one of the semi-permeable membrane tubes (8) defines an outer surface which is arranged closer to an interior part of the flow channel (4, 5) than the outer wall (2) of the flow channel (4, 5) having the opening (7) formed therein, the semi-permeable membrane tube (8) and the outer wall (2) thereby in combination defining a sample collecting volume.

7. A micro-dialysis probe (6) according to any of claims 1-6, further comprising a plug (10) for sealing the first flow channel (4) and the second flow channel (5), said plug (10) forming a wall of the communication opening (9).

8. A micro-dialysis probe (6) according to any of claims 1-7, wherein the separating wall (3) is a substantially plane wall dividing an interior part of the flow tube (1) into the first flow channel (4) and the second flow channel (5).

9. A micro-dialysis probe (6) according to any of claims 1-8, wherein the at least one surface region (19) comprises a layer of substantially non-permeable material applied thereto.

10. A micro-dialysis probe (6) according to claim 9, wherein at least one of the semi-permeable membrane tube(s) (8) comprises two surface regions (19) having a layer of substantially non-permeable material applied thereto, said surface regions (19) being arranged in such a manner that a surface region (20) without substantially non-permeable material applied thereto is arranged between said two surface regions (19).

11. A micro-dialysis probe (6) according to claim 9 or 10, wherein the substantially non-permeable material is a biocompatible material.

12. A micro-dialysis probe (6) according to any of the preceding claims, wherein the at least one non-permeable material forms interface between the edges of the opening and the membrane tube.

13. A method of making a micro-dialysis probe (6), the method comprising the steps of:
- providing a first flow channel (4), a second flow channel (5) and a separating wall (3) arranged in such a manner that it separates the first flow channel (4) and the second flow channel (5),
- providing at least one opening (7) in an outer wall (2) of the first flow channel (4) or the second flow channel (5),
- arranging a semi-permeable membrane tube (8) in each opening (7), where each semi-permeable membrane tube (8) comprises at least one non-permeable section (19), and
- removing material from the separating wall (3), thereby providing a communication opening (9) providing fluid communication between the first flow channel (4) and the second flow channel (5).

14. A method according to claim 13, wherein each non-permeable section (19) forms and area where the semi-permeable membrane (8) is glued or welded into the opening (7).

## Patentansprüche

1. Mikrodialysesonde (6), umfassend:
- eine Strömungsröhre (1), umfassend:
- einen ersten Strömungskanal (4),
- einen zweiten Strömungskanal (5) und
- eine Trennwand (3), die so angeordnet ist, dass sie den ersten Strömungskanal (4) und den zweiten Strömungskanal (5) trennt,
- eine in der Trennwand (3) definierte Kommunikationsöffnung (9) zur Bereitstellung von Fluidkommunikation zwischen dem ersten Strömungskanal (4) und dem zweiten Strömungskanal (5),
**gekennzeichnet durch**
- mindestens eine halbdurchlässige Membranröhre (8), wobei jede halbdurchlässige Membranröhre (8) in einer Öffnung (7) angeordnet ist, die in einer Außenwand (2) des ersten Strömungskanals (4) oder des zweiten Strömungskanals (5) gebildet ist, und
wobei jede halbdurchlässige Membranröhre (8) mindestens einen nichtdurchlässigen Abschnitt (19) umfasst.

2. Mikrodialysesonde (6) nach Anspruch 1, wobei jeder nichtdurchlässige Abschnitt (19) den Bereich bildet, in dem die halbdurchlässige Membran (8) in die Öffnung (7) geklebt oder geschweißt ist.

3. Mikrodialysesonde (6) nach Anspruch 2, umfassend eine erste halbdurchlässige Membranröhre (8a), die in einer in einer Außenwand (2) des ersten Strömungskanals (4) gebildeten Öffnung (7a) angeordnet ist, und eine zweite halbdurchlässige Membranröhre (8b), die in einer Öffnung (7b) des zweiten Strömungskanals (5) angeordnet ist.

4. Mikrodialysesonde (6) nach Anspruch 2 oder 3, wobei die Kommunikationsöffnung (9) an einem Endabschnitt der Sonde (6) angeordnet ist.

5. Mikrodialysesonde (6) nach Anspruch 4, wobei die Kommunikationsöffnung (9) durch Entfernen von Material aus einem Endabschnitt der Trennwand (3) gebildet worden ist.

6. Mikrodialysesonde (6) nach einem der Ansprüche 1 - 15, wobei mindestens eine der halbdurchlässigen Membranröhren (8) eine äußere Fläche definiert, die näher an einem inneren Teil des Strömungskanals (4, 5) angeordnet ist als die Außenwand (2) des Strömungskanals (4, 5) mit der darin gebildeten Öffnung (7), wobei die halbdurchlässige Membranröhre (8) und die Außenwand (2) dadurch in Kombination ein Probennahmevolumen definieren.

7. Mikrodialysesonde (6) nach einem der Ansprüche 1 - 6, ferner umfassend einen Stopfen (10) zum Abdichten des ersten Strömungskanals (4) und des zweiten Strömungskanals (5), wobei der Stopfen (10) eine Wand der Kommunikationsöffnung (9) bildet.

8. Mikrodialysesonde (6) nach einem der Ansprüche 1 - 7, wobei die Trennwand (3) im Wesentlichen eine ebene Wand ist, die einen inneren Teil der Strömungsröhre (1) in den ersten Strömungskanal (4) und den zweiten Strömungskanal (5) teilt.

9. Mikrodialysesonde (6) nach einem der Ansprüche 1 - 8, wobei der mindestens eine Oberflächenbereich (19) eine darauf aufgebrachte Schicht aus im Wesentlichen nichtdurchlässigem Material umfasst.

10. Mikrodialysesonde (6) nach Anspruch 9, wobei mindestens eine der halbdurchlässigen Membranröhren (8) zwei Oberflächenbereiche (19) mit einer darauf aufgebrachten Schicht aus im Wesentlichen nichtdurchlässigem Material umfasst, wobei die Oberflächenbereiche (19) so angeordnet sind, dass ein Oberflächenbereich (20) ohne darauf aufgebrachtem, im Wesentlichen nichtdurchlässigem Material zwischen den beiden Oberflächenbereichen (19) angeordnet ist.

11. Mikrodialysesonde (6) nach Anspruch 9 oder 10, wobei das im Wesentlichen nichtdurchlässige Material ein biologisch kompatibles Material ist.

12. Mikrodialysesonde (6) nach einem der vorhergehenden Ansprüche, wobei das mindestens eine nichtdurchlässige Material eine Schnittstelle zwischen den Rändern der Öffnung und der Membranröhre bildet.

13. Verfahren zur Herstellung einer Mikrodialysesonde (6), umfassend die folgenden Schritte:
- Bereitstellen eines ersten Strömungskanals (4), eines zweiten Strömungskanals (5) und einer Trennwand (3), die so angeordnet ist, dass sie den ersten Strömungskanal (4) und den zweiten Strömungskanal (5) trennt,
- Bereitstellenn mindestens einer Öffnung (7) in einer Außenwand (2) des ersten Strömungskanals (4) oder des zweiten Strömungskanals (5),
- Anordnen einer halbdurchlässigen Membranröhre (8) in jeder Öffnung (7), wobei jede halbdurchlässige Membranröhre (8) mindestens einen nichtdurchlässigen Abschnitt (19) umfasst, und
- Entfernen von Material von der Trennwand (3), wodurch eine Kommunikationsöffnung (9) bereitgestellt wird, die Fluidkommunikation zwischen dem ersten Strömungskanal (4) und dem zweiten Strömungskanal (5) bereitstellt.

14. Verfahren nach Anspruch 13, wobei jeder nichtdurchlässige Abschnitt (19) einen Bereich bildet, in dem die halbdurchlässige Membran (8) in die Öffnung (7) geklebt oder geschweißt ist.

## Revendications

1. Sonde de micro-dialyse (6) comprenant :
- un tube d'écoulement (1), comprenant :
- un premier canal d'écoulement (4),
- un deuxième canal d'écoulement (5), et
- une paroi de séparation (3) disposée de manière à séparer le premier canal d'écoulement (4) et le deuxième canal d'écoulement (5),
- une ouverture de communication (9) définie dans la paroi de séparation (3) pour assurer une communication fluidique entre le premier canal d'écoulement (4) et le deuxième canal d'écoulement (5) ; **caractérisée par**
- au moins un tube à membrane semi-perméable (8), chaque tube à membrane semi-perméable (8) étant disposé dans une ouverture (7) formée dans une paroi extérieure (2) du premier canal d'écoulement (4) ou du deuxième canal d'écoulement (5), et
chaque tube à membrane semi-perméable (8) comprenant au moins une section non perméable (19).

2. Sonde de micro-dialyse (6) selon la revendication 1, dans laquelle chaque section non perméable (19) forme la zone dans laquelle la membrane semi-perméable (8) est collée ou soudée dans l'ouverture (7).

3. Sonde de micro-dialyse (6) selon la revendication 2, comprenant au moins un premier tube à membrane semi-perméable (8a) agencé dans une ouverture (7a) formée dans une paroi extérieure (2) du premier canal d'écoulement (4) et un deuxième tube à membrane semi-perméable (8) agencé dans une ouverture (7b) du deuxième canal d'écoulement (5).

4. Sonde de micro-dialyse (6) selon la revendication 2 ou 3, dans laquelle l'ouverture de communication (9) est disposée au niveau d'une portion d'extrémité de la sonde (6).

5. Sonde de micro-dialyse (6) selon la revendication 4, dans laquelle l'ouverture de communication (9) a été formée en enlevant de la matière d'une portion d'extrémité de la paroi de séparation (3).

6. Sonde de micro-dialyse (6) selon l'une quelconque des revendications 1 à 15, dans laquelle au moins l'un des tubes à membrane semi-perméable (8) définit une surface extérieure qui est agencée plus près d'une partie intérieure du canal d'écoulement (4, 5) que la paroi extérieure (2) du canal d'écoulement (4, 5) dans lequel est formée l'ouverture (7), le tube à membrane semi-perméable (8) et la paroi extérieure (2) définissant ainsi en combinaison un volume de collecte d'échantillon.

7. Sonde de micro-dialyse (6) selon l'une quelconque des revendications 1 à 6, comprenant en outre un bouchon (10) pour sceller le premier canal d'écoulement (4) et le deuxième canal d'écoulement (5), ledit bouchon (10) formant une paroi de l'ouverture de communication (9).

8. Sonde de micro-dialyse (6) selon l'une quelconque des revendications 1 à 7, dans laquelle la paroi de séparation (3) est une paroi substantiellement plane divisant une partie intérieure du tube d'écoulement (1) en le premier canal d'écoulement (4) et le deuxième canal d'écoulement (5).

9. Sonde de micro-dialyse (6) selon l'une quelconque des revendications 1 à 8, dans laquelle l'au moins une région de surface (19) comprend une couche de matériau substantiellement non perméable appliquée sur celle-ci.

10. Sonde de micro-dialyse (6) selon la revendication 9, dans laquelle au moins l'un des tubes à membrane semi-perméable (8) comprend deux régions de surface (19) ayant une couche de matériau substantiellement non perméable appliquée sur celles-ci, lesdites régions de surface (19) étant disposées de manière à ce qu'une région de surface (20) sur laquelle aucun matériau substantiellement non perméable n'est appliqué soit agencée entre lesdites deux régions de surface (19).

11. Sonde de micro-dialyse (6) selon la revendication 9 ou 10, dans laquelle le matériau substantiellement non perméable est un matériau biocompatible.

12. Sonde de micro-dialyse (6) selon l'une quelconque des revendications précédentes, dans laquelle l'au moins un matériau non perméable forme une interface entre les bords de l'ouverture et le tube à membrane.

13. Procédé de fabrication d'une sonde de micro-dialyse (6), le procédé comprenant les étapes suivantes :
- fournir un premier canal d'écoulement (4), un deuxième canal d'écoulement (5) et une paroi de séparation (3) agencée de telle sorte qu'elle sépare le premier canal d'écoulement (4) et le deuxième canal d'écoulement (5),
- fournir au moins une ouverture (7) dans une paroi extérieure (2) du premier canal d'écoulement (4) ou du deuxième canal d'écoulement (5),
- agencer un tube à membrane semi-perméable (8) dans chaque ouverture (7), chaque tube à membrane semi-perméable (8) comprenant au moins une section non perméable (19), et
- enlever du matériau de la paroi de séparation (3), pour ainsi fournir une ouverture de communication (9) assurant une communication fluidique entre le premier canal d'écoulement (4) et le deuxième canal d'écoulement (5).

14. Procédé selon la revendication 13, dans lequel chaque section non perméable (19) forme une zone dans laquelle la membrane semi-perméable (8) est collée ou soudée dans l'ouverture (7).
